# EUROPÄISCHE PATENTSCHRIFT

(11) **EP 3 574 833 B1**
(45) Veröffentlichungstag und Bekanntmachung des Hinweises auf die Patenterteilung: **20.10.2021**
(21) Anmeldenummer: 18174897.1
(22) Anmeldetag: 29.05.2018
(51) Int. Cl.: A61B 6/00, H05G 1/26, H05G 1/54, G16H 40/40

(54) **VERFAHREN ZUR STATUSÜBERWACHUNG EINER EINE RÖNTGENSTRAHLENQUELLE UMFASSENDEN RÖNTGENSTRAHLERANORDNUNG FÜR EINE RÖNTGENEINRICHTUNG, RÖNTGENSTRAHLERANORDNUNG, COMPUTERPROGRAMM UND ELEKTRONISCH LESBARER DATENTRÄGER**
METHOD FOR MONITORING THE STATUS OF A X-RAY SOURCE-COMPRISING A X-RAY RADIATOR ARRANGEMENT FOR AN X-RAY DEVICE, X-RAY RADIATOR ARRANGEMENT, COMPUTER PROGRAM AND ELECTRONICALLY READABLE DATA CARRIER
PROCÉDÉ DE SURVEILLANCE DE L'ÉTAT D'UN DISPOSITIF FORMANT ENSEMBLE RADIOGÈNE À RAYONNEMENT X COMPORTANT UNE SOURCE DE RAYONS X POUR UN DISPOSITIF À RAYON X, DISPOSITIF FORMANT ENSEMBLE RADIOGÈNE À RAYONNEMENT X, PROGRAMME INFORMATIQUE ET SUPPORT DE DONNÉES LISIBLE PAR VOIE ÉLECTRONIQUE

(43) Veröffentlichungstag der Anmeldung: 04.12.2019
(73) Patentinhaber: Siemens Healthcare GmbH, 91052 Erlangen (DE)
(72) Erfinder: Haider, Sultan, 91052 Erlangen (DE); Freudenberger, Jörg, 90562 Kalchreuth (DE)

(56) Entgegenhaltungen:
- EP-A1- 2 210 560
- DE-A1- 10 011 294
- DE-A1-102011 006 125
- DE-B3- 10 338 693
- US-A1- 2018 061 207

## Beschreibung

Die Erfindung betrifft ein Verfahren zur Statusüberwachung einer eine Röntgenstrahlenquelle umfassenden Röntgenstrahleranordnung für eine Röntgeneinrichtung. Daneben betrifft die Erfindung eine Röntgenstrahleranordnung, ein Computerprogramm und einen elektronisch lesbaren Datenträger.

Röntgeneinrichtungen verschiedener Art werden in der medizinischen Bildgebung als etablierte Bildgebungsmodalitäten eingesetzt. Beispielsweise sind C-Bogen-Röntgeneinrichtungen, Computertomographieeinrichtungen und andere Arten von Röntgeneinrichtungen bekannt. Jede Röntgeneinrichtung weist eine Röntgenstrahlenquelle, beispielsweise eine Röntgenröhre, und einen Röntgendetektor auf, um Röntgenbilder anhand der den Bildgebungsbereich des Patienten durchquerenden Röntgenstrahlung, die von der Röntgenstrahlenquelle erzeugt wird, aufzunehmen.

Moderne Röntgenstrahlenquellen, die gemeinsam mit einem Gehäuse und/oder sonstigen Befestigungsmitteln eine Röntgenstrahleranordnung (bzw. Röntgenstrahlervorrichtung) bilden, sind empfindliche Einrichtungen und gleichzeitig Verschleißteile der Röntgeneinrichtung, das bedeutet, eine Röntgenstrahlenquelle hat eine bestimmte Lebensdauer, für die sie in der Röntgeneinrichtung eingesetzt werden kann. Auch die Erwärmung der Röntgenstrahlenquelle bei ihrem Betrieb sowie sonstige Effekte können zu Einschränkungen bei der Benutzung führen, beispielsweise die Benutzungsdauer bei einer bestimmten Art von Untersuchung einschränken. Hierin gehen auch Betriebsparameter der Röntgenstrahlenquelle ein, beispielsweise eine Röhrenspannung bei einer Röntgenröhre als Röntgenstrahlungsquelle.

Mithin bestehen für den Benutzer der Röntgenstrahleranordnung während des Einsatzes der Röntgenstrahlenquelle verschiedenste Fragestellungen, beispielsweise, den Status der Röntgenstrahlenquelle feststellen zu können, insbesondere, wie lange sie bei einer bestimmten Art von Untersuchungen mit einer bestimmten Frequenz eingesetzt werden kann, wie die Röntgenstrahlereinrichtung für Höchstqualität betrieben werden soll, wie Wartungsmaßnahmen durchgeführt werden können, beispielsweise ein Austausch der Röntgenstrahlenquelle und der gesamten Röntgenstrahleranordnung, und dergleichen. Nachdem es sich bei der Röntgenstrahlenquelle um ein empfindliches technisches Gerät handelt, ist zudem bei deren Transport eine besondere Vorsicht geboten.

Derartige Informationen werden heutzutage üblicherweise durch Handbücher oder Schulungen an entsprechende Serviceingenieure oder sonstige Systemnutzer weitergegeben, was teuer und zeitaufwendig ist. Informationen über den Status der Röntgenstrahlenquelle und Unterstützung während der Benutzung der Röntgenstrahlenquelle, insbesondere einer Untersuchung, sind an der Röntgenstrahlenquelle bzw. Röntgenstrahleranordnung heutzutage nicht verfügbar.

Für manche Aspekte des Status der Röntgenstrahlenquellen wurde vorgeschlagen, Datamining-Tools einzusetzen, die aus bestimmten Parametern der Röntgenstrahlenquelle Prädiktionen über die zukünftige Nutzung ableiten und Benutzern zur Verfügung stellen können. Bei derartigen Datamining-Ansätzen sind jedoch meist Verträge zwischen Hersteller und Benutzer erforderlich.

Eine indirekte Ableitung von Statusinformationen einer Röntgenstrahlenquelle ist zudem über spezielle Service-Softwareanwendungen der Röntgeneinrichtung möglich, in der die Röntgenstrahleranordnung verwendet wird. Allerdings finden sich die notwendigen Informationen dann eher verteilt und es handelt sich um eine äußerst komplexe Softwareapplikation, so dass es einen großen Aufwand für einen Benutzer darstellt, eine Statusinformation zu der Röntgenstrahlenquelle zu erhalten. An der Röntgenstrahleranordnung selbst sind meist nur Aufkleber vorhanden, die Gegenstand- und Herstellerinformationen zu der Röntgenstrahlerquelle enthalten können, beispielsweise den Hersteller, eine Seriennummer, technische Spezifikationen und dergleichen. Über den aktuellen Zustand, also den Status, der Röntgenstrahlenquelle sind mithin keine Informationen vorhanden.

EP 2 210 560 A1 offenbart ein System und eine Methode zur Vorhersage eines Ausfalls eines Bildgebungssystems, welche eine Strahlenquelle mit einer Röntgenstrahleranordnung umfasst. Aus DE 100 11 294 A1 ist eine Röntgenröhre mit einem thermionischen Emitter und einer Warneinrichtung zur Signalisierung eines bevorstehenden Ausfalls des thermionischen Emitters bekannt. In DE 10 2011 006 125 A1 ist ein Geräuschüberwachungssystem zur Überwachung und Regelung von Lagergeräuschen eines Gleitlagers eines Röntgenstrahlers beschrieben. DE 103 38 693 B3 betrifft u.a. ein Verfahren zum Abschätzen der Restlebensdauer eines in Betrieb befindlichen und in einem Röntgengerät eingebauten Röntgenstrahlers. Eine Möglichkeit zur Abschätzung eines Beschädigungsgrades von verderblicher Ware während dessen Transports offenbart US 2018 / 0 061 207 A1.

Der Erfindung liegt daher die Aufgabe zugrunde, eine Möglichkeit zur verbesserten Information eines Benutzers über den Röntgenstrahlenquellenzustand anzugeben.

Diese Aufgabe wird gelöst durch ein Verfahren mit den Merkmalen des Anspruchs 1, eine Röntgenstrahleranordnung mit den Merkmalen des Anspruchs 11, ein Computerprogramm mit den Merkmalen des Anspruchs 12 und einen elektronisch lesbaren Datenträger mit den Merkmalen des Anspruchs 13.

Ein erfindungsgemäßes Verfahren zur Statusüberwachung einer eine Röntgenstrahlenquelle umfassenden Röntgenstrahleranordnung für eine Röntgeneinrichtung umfasst folgende Schritte:
- Ermitteln einer den aktuellen Zustand der Röntgenstrahlenquelle beschreibenden Eingangsinformation durch wenigstens ein Ermittlungsmittel,
- Ableiten einer an einen Benutzer auszugebenden Statusinformation unter Verwendung der Eingangsinformation mittels eines Verarbeitungsmittels, und
- Anzeigen der Statusinformation auf einer Anzeigevorrichtung, insbesondere einem Display,
   dadurch gekennzeichnet, dass als Eingangsinformation eine Transportbedingungen während eines Transports beschreibende Bedingungsinformation verwendet wird.

Es wird mithin vorgeschlagen, eine Anzeigevorrichtung, insbesondere ein Display, dediziert zur Ausgabe von Statusinformationen bezüglich der Röntgenstrahleranordnung zu nutzen und somit der Röntgenstrahleranordnung mit ihrer Röntgenstrahlenquelle wenigstens zeitweise zuzuordnen. Dabei ist die Anzeigevorrichtung mit besonderem Vorteil dauerhaft der Röntgenstrahlenquelle zugeordnet, bildet also insbesondere sogar einen Teil der Röntgenstrahleranordnung. Das bedeutet, mit besonderem Vorteil kann eine in, an oder benachbart der Röntgenstrahlenanordnung befestigte und/oder dieser zugehörige Anzeigevorrichtung, insbesondere ein Display, verwendet werden.

Alternativ oder zusätzlich hierzu sind auch andere Ausgestaltungen denkbar, in denen beispielsweise externe Anzeigevorrichtungen genutzt werden, sei es beispielsweise ohnehin an der Röntgeneinrichtung vorgesehene Anzeigevorrichtungen oder beispielsweise unter Nutzung einer geeigneten SoftwareApplikation ansprechbare Anzeigevorrichtungen an Mobilgeräten eines Benutzers, beispielsweise einem Smartphone. In diesem Fall kann ein Computerprogramm zur Umsetzung des Verfahrens beispielsweise eine herunterladbare Software-Applikation ("App") für das Mobilgerät umfassen, welches Kommunikationsverbindungen zu Quellen von Eingangsinformationen herstellen kann, beispielsweise zu einer Steuereinrichtung der Röntgeneinrichtung und/oder einer externen Servereinrichtung, wobei die herunterladbare Software-Applikation dann auch selbst das Ermittlungsmittel und das Verarbeitungsmittel realisieren kann. Selbstverständlich sind auch verteilte Ausführungen des erfindungsgemäßen Verfahrens oder anderweitige Umsetzungen möglich, beispielsweise durch ein Webseitenportal, welches beispielsweise mit der Röntgeneinrichtung und/oder einer externen Servereinrichtung, auf der die Eingangsinformationen vorliegen, kommuniziert und diese entsprechend verarbeitet. Ersichtlich sind verschiedene konkrete Ausgestaltungen gegeben, um ein derartiges "Statusdisplay" umzusetzen, wobei, wie bereits erwähnt, in der bevorzugten Ausgestaltung die Röntgenstrahleranordnung um eine Anzeigevorrichtung, insbesondere ein Display, sowie das Verarbeitungsmittel und das wenigstens eine Ermittlungsmittel erweitert wird. Dabei wird ein aktives bzw. bevorzugt sogar interaktives Display zu der bislang passiven Komponente Röntgenstrahlenquelle hinzugefügt.

Durch die neu gegebenen Möglichkeiten eröffnen sich dem das erfindungsgemäße Verfahren nutzenden Hersteller verschiedenste Vorteile, insbesondere, indem benutzerfreundlichere Röntgenstrahlenquellen, insbesondere Röntgenröhren, geschaffen werden und die in der Interaktion mit dem Benutzer bislang problematischen Punkte hinsichtlich dessen Information gezielt adressiert werden können.

Konkret kann hierbei die Eingangsinformation wenigstens teilweise von einer Steuereinrichtung der Röntgeneinrichtung und/oder einer Steuereinheit der Röntgenstrahleranordnung und/oder einer externen Servereinrichtung abgefragt werden und/oder wenigstens teilweise durch wenigstens einen der Röntgenstrahleranordnung zugeordnetes oder dieser zugehöriges Erfassungsmittel, insbesondere einen Sensor, erfasst werden. Ein Teil nützlicher Eingangsinformationen wird in modernen Röntgeneinrichtungen in einigen Ausgestaltungen bereits erfasst, so dass auf diese entsprechend erfasste Information in der Steuereinrichtung der Röntgeneinrichtung zweckmäßig zugegriffen werden kann. Mit besonderem Vorteil werden, insbesondere zur Ermittlung von den aktuellen Betriebszustand bzw. Allgemeinzustand der Röntgenstrahlenquelle betreffenden Eingangsinformationen wie deren Temperatur und dergleichen, Sensoren oder sonstige Erfassungsmittel eingesetzt, die ihre Erfassungstätigkeit bevorzugt unmittelbar an der Röntgenstrahlenquelle bzw. der Röntgenstrahleranordnung durchführen.

Als Einfangsinformationen können zusätzlich zu der die Transportbedingungen beschreibenden Bedingungsinformation eine die Nutzung der Röntgenstrahlenquelle in der Vergangenheit und/oder für eine unmittelbar vorstehende Messung beschreibende Nutzungsinformationen und/oder eine das Alter der Röntgenstrahlenquelle beschreibende Altersinformation und/oder Lagerungsbedingungen beschreibende Bedingungsinformationen erfasst werden. Dabei können sich Bedingungsinformationen selbstverständlich auch auf Betriebsbedingungen beziehen und beispielsweise eine Temperatur der Röntgenstrahlenquelle und/oder eine Luftfeuchtigkeit der Umgebung der Röntgenstrahlenquelle und/oder in die Röntgenstrahleranordnung induzierte Vibrationen und/oder ein Öldruck in der Röntgenstrahlenquelle verwendet werden.

Gerade die Nutzung von Bedingungsinformation als Eingangsinformation lässt insbesondere auch eine Transportüberwachung der Röntgenstrahlenquelle mittels des erfindungsgemäßen Verfahrens zu. So kann konkret vorgesehen sein, dass als Statusinformation bei Vorliegen des Transportvorgangs der Röntgenstrahleranordnung eine Transportüberwachungsinformation ermittelt wird. Wie bereits erwähnt wurde, handelt es sich bei einer Röntgenstrahlenquelle, beispielsweise einer Röntgenröhre, um eine empfindliche technische Vorrichtung, deren Lebensdauer und/oder Einsatzbereitschaft stark von den äußeren Bedingungen abhängig sein kann, denen sie ausgesetzt wird. Beispielsweise sind Röntgenröhren bekannt, die idealerweise in einem bestimmten Temperaturbereich bzw. in einem speziellen Luftfeuchtigkeitsbereich gehalten werden sollten.

Zudem sollten Vibrationen gegebenenfalls innerhalb eines erlaubten Bereichs gehalten werden. Derartige Sollbereiche können durch Sollwerte beschrieben werden, so dass es besonders vorteilhaft ist, wenn die Ermittlung der Transportüberwachungsfunktion den Vergleich von Eingangsinformationen mit Sollwerten für den Transport umfasst. Dadurch wird es insbesondere ermöglicht, bei außerhalb eines durch Sollwerte beschriebenen Sollbereichs liegenden Transportbedingungen, die Statusinformation als eine Warnmeldung auszugeben. Es sind also konkrete Ausführungsbeispiele denkbar, in denen die Anzeigevorrichtung beispielsweise kontinuierlich die Transportüberwachungsinformation darstellt, beispielsweise in grün, während die Röntgenstrahleranordnung mit der Röntgenstrahlungsquelle transportiert wird, während dann, wenn die Eingangsinformationen außerhalb der Sollbereiche liegende Werte anzeigen, eine nachhaltigere Warnung ausgegeben werden kann, insbesondere unter zusätzlicher akustischer und/oder haptischer und/oder optischer Ausgabe eines Warnsignals. Dabei sei darauf hingewiesen, dass die Transportüberwachung besonders vorteilhaft bei einem der Röntgenstrahleranordnung zugehörigen Display als Anzeigevorrichtung umgesetzt werden kann, welches insbesondere auch beim Transport der Röntgenstrahleranordnung im Blickfeld der transportierenden Person bzw. für diese einsehbar positionierbar ist und dergleichen.

In dieser Ausgestaltung ist es im Übrigen auch zweckmäßig, Transportvorgänge zu protokollieren, beispielsweise, indem die Transportüberwachungsinformation abgespeichert wird oder zumindest Warnereignisse während eines Transportvorgangs protokolliert und beispielsweise in einem Speicher des Verarbeitungsmittels bzw. einer das Verarbeitungsmittel realisierenden Steuereinheit abgelegt werden, um dort später abgerufen zu werden. Liegt eine Kommunikationsverbindung des hier beschriebenen Statusüberwachungssystems zu einer externen Servereinrichtung, beispielsweise in das Internet, ohnehin vor, lassen sich selbstverständlich bei einem Transportvorgang entstehende Warnungen und dergleichen auch unmittelbar an die externe Servereinrichtung mithin übertragen, dort protokollieren und/oder zur dortigen Ausgabe eines Alarms auswerten.

Dabei sei an dieser Stelle noch angemerkt, dass die Statusinformation nicht nur während des Transports zweckmäßig zugänglich gemacht werden kann, sondern, insbesondere unter Nutzung unterschiedlicher Anzeigevorrichtungen und/oder unterschiedlicher Statusinformationen, nützliche Statusinformationen auch in weiteren Betriebszuständen der Röntgenstrahlenquelle einem Benutzer ausgegeben werden können, beispielsweise während der Installation, während der Kalibirierung, während der Benutzung, in Fehlerfällen, während Wartungsvorgängen, während der Deinstallation und/oder während eines Refurbishment.

Mithin können abhängig von einer aktuellen Nutzungsphase unterschiedliche Statusinformationen bereitgestellt werden, wobei im Fall einer interaktiven Anzeigevorrichtung, die also eine Eingabevorrichtung umfasst oder der eine Eingabevorrichtung zugeordnet ist, auch eine Benutzereingabe einen Einfluss auf die konkret dargestellte Statusinformation haben kann, nachdem ein Benutzer beispielsweise eine Nutzerphase wählen kann, beispielsweise "Transport", "Installation", "Kalibrierung", und dergleichen.

Neben des Beispiels der Transportüberwachungsinformation können noch eine Vielzahl anderer nützlicher Statusinformationen erzeugt und ausgegeben werden.

Eine besonders vorteilhafte Weiterbildung der vorliegenden Erfindung sieht vor, dass als weitere Statusinformation eine die zukünftige Nutzbarkeit der Röntgenstrahlenquelle beschreibende Prädiktionsinformation ermittelt wird. Eine derartige Prädiktionsinformation kann beispielsweise eine erwartete zukünftige Lebensspanne sein, die in Abhängigkeit von Daten über die vergangene Nutzung der Röntgenstrahlenquelle ermittelt werden kann, beispielsweise durchgeführte Untersuchungen, Anzahl der durchgeführten Untersuchungen bzw. Art der durchgeführten Untersuchungen und dergleichen. Auch Informationen über aktuell durchzuführende Untersuchungen, beispielsweise ein aktuelles Messprotokoll, können eingehen, wobei dem Verarbeitungsmittel, beispielsweise auch als Eingangsinformationen, Verschleißinformationen bezüglich der Röntgenstrahlenquelle vorliegen können, denen beispielsweise auch vorangegangene Untersuchungen zur Lebensdauer und/oder Eigenschaften bezüglich der Röntgenstrahlenquelle bzw. einer vergleichbaren Röntgenstrahlenquelle unter Gebrauch zugrunde liegen können.

Zur Statusüberwachung bei der Installation kann beispielsweise eine Information über gesteckte Anschlüsse in der Statusinformation enthalten sein. Während der Benutzung zur Bildgebung können beispielsweise aktuelle Betriebsparameter der Röntgenstrahlenquelle angezeigt werden, genau wie Informationen zum Wärmemanagement der Röntgenstrahlenquelle, die sich insbesondere bei länger andauernden Untersuchungen als nützlich erweisen. Dies ist insbesondere dann zweckmäßig, wenn die Röntgenstrahlenquelle nicht sichtbar verbaut ist, ihr mithin ihre Temperatur nicht unmittelbar angesehen werden kann und dergleichen.

Zweckmäßigerweise kann in Abhängigkeit von einer Benutzereingabe über eine der Anzeigevorrichtung und/oder der Röntgenstrahleranordnung und/oder der Röntgeneinrichtung zugeordnete und/oder mit der Anzeigevorrichtung in Baueinheit realisierte Eingabevorrichtung und/oder von der Erfüllung einer insbesondere wenigstens eine Eingabeinformation auswertenden Ausgabebedingung die Statusinformation ausgegeben werden. Das bedeutet, Statusinformationen können beispielsweise durch entsprechende Benutzereingaben über eine Eingabevorrichtung angefordert werden, wobei es jedoch auch möglich ist, beispielsweise durch die Erfüllung von Ausgabebedingungen als Triggerereignis automatisch zu sinnvollen Zeitpunkten Statusinformationen auszugeben, die ein Benutzer zu dieser Zeit benötigen könnte. So kann beispielsweise automatisch bei Beginn der Röntgenstrahlenerzeugung durch die Röntgenstrahlenquelle begonnen werden, die aktuellen Betriebsparameter und/oder Informationen zum Wärmemanagement als Statusinformationen auszugeben, welche bis zum Ende der aktuellen Betriebsphase angezeigt werden können. Liegt beispielsweise ein Fehlerzustand vor, kann eine entsprechende Ausgabebedingung dafür sorgen, dass die Anzeigevorrichtung gegebenenfalls aktiviert wird und als Statusinformation Informationen zu dem aktuell vorliegenden Fehler ausgibt. Über die Benutzereingaben kann auch beispielsweise eine Art Menü realisiert werden, in dem ein Benutzer zwischen unterschiedlichen Arten von Statusinformationen wechseln kann.

Besonders zweckmäßig ist es, dass zusätzlich und/oder nach einer Benutzereingabe und/oder Erfüllung einer weiteren Ausgabebedingung wenigstens eine weitere, auf die Röntgenstrahlenquelle bezogene, insbesondere ebenso abhängig von der Eingangsinformation ermittelte Ausgabeinformation auf der Anzeigevorrichtung ausgegeben wird. Das bedeutet, über eine Statusinformation hinaus kann die Anzeigevorrichtung auch weitere nützliche Ausgabeinformationen bezüglich der Röntgenstrahlenquelle an einen Benutzer weitergeben. Beispielsweise kann als Ausgabeinformation eine einen Wartungsvorgang beschreibende Wartungsinformation und/oder eine Handbuchinformation und/oder eine technische Eigenschaft und/oder Benennung der Röntgenstrahlenquelle beschreibende Gegenstandsinformation und/oder eine den Hersteller der Röntgenstrahlenquelle beschreibende Herstellerinformation, insbesondere ein Logo, und/oder eine auf einer aktuellen mit der Röntgenstrahlenquelle durchzuführende Untersuchung bezogene Untersuchungsinformation und/oder eine die Bedienung der Röntgenstrahlenquelle und/oder der Röntgeneinrichtung erläuternde Lehrinformation verwendet werden.

Gerade in Verbindung mit Statusinformationen sind Wartungsinformationen besonders geeignete Ausgabeinformationen, da dann einem Benutzer beispielsweise Wartungsschritte mittels der Anzeigevorrichtung erklärt und nähergebracht werden können. Insbesondere bei räumlich der Röntgenstrahleranordnung zugeordnet angeordneten Anzeigevorrichtungen kann der Benutzer dann beispielsweise an einer Röntgenstrahleranordnung arbeiten und erhält in unmittelbarem Kontext nützliche Informationen über die Anzeigevorrichtung. Unter zusätzlicher Nutzung einer Eingabevorrichtung, beispielsweise eines entsprechenden Rasters, kann der Benutzer beispielsweise schrittweise durch einen Wartungsprozess, beispielsweise einen Austausch von Komponenten, geführt werden, wobei die entsprechende Wartungsinformation bevorzugt um entsprechend passende, nützliche Statusinformationen ergänzt wird, beispielsweise Hinweise auf offene Wartungsklappen, vorliegende Anschlüsse, vorliegende Fehlerzustände und dergleichen.

Auf der Anzeigevorrichtung können auch Gegenstandsinformationen dargestellt werden, beispielsweise die Art der Röntgenstrahlenquelle, technische Spezifikationen der Röntgenstrahlenquelle, der Name des Herstellers, der Herstellungsort, die Seriennummer, ein Logo oder sonstige Gegenstands/Herstellerinformationen. Ein Logo des Herstellers bzw. des Typs der Röntgenstrahlungsquelle kann beispielsweise auch als eine Art "Bildschirmschoner" verwendet werden, wenn gerade keine Statusinformationen anderer Art wiedergegeben werden.

Wird die Röntgenstrahlenquelle gerade für eine Untersuchung verwendet, können diesbezügliche Untersuchungsinformationen ausgegeben werden, beispielsweise ein zu untersuchender Körperbereich, ein gewähltes Messprotokoll, eine Untersuchungsdauer, Informationen zu anderen Komponenten der Röntgeneinrichtung, Informationen zum Patienten und dergleichen.

In einer besonders nützlichen Ausgestaltung kann das Statusüberwachungssystem auch einen Lehr-Betriebsmodus aufweisen, in dem die Anzeigevorrichtung genutzt wird, um Lehrinformationen hinsichtlich der Bedienung der Röntgenstrahlenquelle und/oder der Röntgeneinrichtung auszugeben. Ein solcher LehrBetriebsmodus informiert/lehrt die Benutzer über verschiedenste mit der Röntgenstrahlenquelle in Verbindung stehende Umstände, beispielsweise Röntgenphysik, Betriebsparameter der Röntgenstrahlenquelle, Funktionalitäten der Röntgenstrahlenquelle, Anwendungsprofile und dergleichen.

Wie bereits angedeutet wurde, kann es im Rahmen der vorliegenden Erfindung zweckmäßig sein, mehrere Anzeigevorrichtungen zu verwenden, auf denen insbesondere unterschiedliche Statusinformationen ausgegeben werden und/oder die zu unterschiedlichen Zeitpunkten betrieben werden. Beispielsweise können unterschiedliche Nutzungsphasen umfassend Transport, Installation, Kalibrierung, Bildgebung, Wartung, Ausbau und dergleichen jeweils eine Anzeigevorrichtung und/oder eine Statusinformation zugeordnet sein.

Besonders bevorzugt ist es im Rahmen der vorliegenden Erfindung, wie bereits erwähnt, eine an oder benachbart der Röntgenstrahleranordnung befestigte und/oder dieser zugehörige Anzeigevorrichtung, insbesondere ein Display, zu verwenden. Für eine derartige Anzeigevorrichtung bietet sich besonders sogenanntes elektronisches Papier an. Dabei kann beispielsweise elektrophoretische Tinte (auch als elektronische Tinte bezeichnet) auf einen Träger, beispielsweise eine Plastikfolie, auflaminiert werden und dann mit einer entsprechenden Elektronik verbunden werden, um ein Display aus elektronischem Papier zu schaffen. Solches elektronische Papier eignet sich besonders für die Anbringung, da es insbesondere auch flexibel vorliegen kann.

Bei an der Röntgenstrahleranordnung selbst vorgesehenen Anzeigevorrichtungen sieht eine weitere zweckmäßige Ausgestaltung vor, dass als Statusinformation und/oder weitere Ausgabeinformationen ein maschinenlesbarer Code, der auf eine andere Informationsquelle, insbesondere im Internet, verweist, ausgegeben wird. Bei dem maschinenlesbaren Code kann es sich insbesondere um einen QR-Code handeln, der im Übrigen auch durch elektronisches Papier vorteilhaft angezeigt werden kann. Ein solcher maschinenlesbarer Code kann nun beispielsweise mit einem Mobilgerät des Benutzers ausgelesen werden und genutzt werden, um auf die weitere Informationsquelle zuzugreifen.

Beispielsweise ist es in einer konkreten Ausgestaltung denkbar, einen QR-Code auszugeben, der einen Servicetechniker zu Informationsquellen weiterleitet, die für seine Tätigkeit nützlich sind, beispielsweise Webseiten, die spezifische Informationen, wie Installationsanweisungen zeigen, Ersatzteillogistik, kompatible Generatoren und Schnittstellen, Kontaktdaten zu einem Support zweiter Stufe und dergleichen.

Im Kontext der röntgenstrahleranordnungsseitigen Bereitstellung der Anzeigevorrichtung ist es im Übrigen auch bevorzugt, wenn wenigstens teilweise auch das Ermittlungsmittel und/oder das Verarbeitungsmittel, insbesondere als eine Steuereinheit, einen Teil der Röntgenstrahleranordnung bilden. Auf diese Weise ist die Röntgenstrahleranordnung mithin mit einer Eigenintelligenz versehen, die für den Benutzer nützliche Informationen bereitstellt, die bei den passiven Röntgenstrahlungsquellen, die bislang bekannt waren, nicht bereitstanden.

Bei Abruf wenigstens eines Teils der Eingabeinformation von einem zum Ermittlungsmittel externen Kommunikationspartner und/oder bei Verwendung einer zum Verarbeitungsmittel externen Anzeigevorrichtung kann wenigstens teilweise eine drahtlose Kommunikationsverbindung, insbesondere Bluetooth, und/oder NFC, verwendet werden. Dies ist insbesondere auch für Steuereinheiten zweckmäßig, die seitens der Röntgenstrahleranordnung vorgesehen sind und das Ermittlungsmittel und das Verarbeitungsmittel realisieren. Dann kann eine Kommunikationsschnittstelle gegeben sein, die sich nicht auf Nahbereichskommunikation wie Bluetooth und/oder NFC beschränken muss, sondern auch drahtlose Kommunikation mit weiter entfernten Servereinrichtungen, beispielsweise Servereinrichtungen des Internets, erlauben kann. Beispiele für solche weitergehenden Schnittstellen umfassen WLAN-Schnittstellen und/oder Mobilfunk-Schnittstellen. Auch über Kommunikationsstandards wie Bluetooth und dergleichen ist es jedoch selbstverständlich möglich, mittelbar, beispielsweise über eine Steuereinrichtung der Röntgeneinrichtung, auf das Internet oder andere Netzwerke zuzugreifen, um die Eingabeinformation abrufen zu können und/oder externe Anzeigevorrichtungen ansprechen zu können.

Als externe, bevorzugt zusätzlich zu verwendende Anzeigevorrichtung kann auch eine Anzeigevorrichtung eines Mobilgeräts eines Benutzers verwendet werden. Beispielsweise können Statusinformation und/oder weitere Ausgabeinformationen auch wenigstens teilweise auf einem Mobilgerät des Benutzers, beispielsweise einem Smartphone, ausgegeben werden; wie bereits erwähnt, ist es jedoch auch denkbar, dass Statusüberwachungssystem komplett auf einem Mobilgerät des Benutzers zu realisieren, beispielsweise durch eine entsprechende SoftwareApplikation, die das Verarbeitungsmittel und das Ermittlungsmittel realisiert.

Es sei an dieser Stelle noch darauf hingewiesen, dass sich auch unabhängig von Mobilgeräten Touchscreens als geeignete Displays bzw. Anzeigevorrichtungen im Rahmen der vorliegenden Erfindung erwiesen haben.

In einer Weiterbildung der vorliegenden Erfindung kann vorgesehen sein, dass bei Vorliegen einer Freischaltinformation und einer Abrufbenutzereingabe über eine der Anzeigevorrichtung zugeordnete Eingabevorrichtung wenigstens eine Servicedienstleistung abrufen und eine auf die Servicedienstleistung bezogene Ausgabeinformation auf der Anzeigevorrichtung dargestellt wird. Insbesondere dann, wenn eine Verbindung zu einer externen Servereinrichtung besteht, kann es im Rahmen der vorliegenden Erfindung mithin auch ermöglicht werden, zusätzliche Services bereitzustellen, falls eine entsprechende vertragliche Grundlage vorliegt, die durch die Freischaltinformation beschrieben wird. Derartige zusätzliche Services können beispielsweise Selbstreparatur, Ausfallvorhersagen, Refurbishment-Optionen und dergleichen umfassen.

Neben dem Verfahren betrifft die vorliegende Erfindung auch eine Röntgenstrahleranordnung, aufweisend
- eine Röntgenstrahlenquelle,
- eine Steuereinheit mit einem Ermittlungsmittel zum Ermitteln einer den aktuellen Zustand der Röntgenstrahlenquelle beschreibenden Eingangsinformation und einem Verarbeitungsmittel zum Ableiten einer an einen Benutzer auszugebenden Statusinformation unter Verwendung der Eingangsinformation, und
- eine von der Steuereinheit ansteuerbare Anzeigevorrichtung zur Anzeige der Statusinformation,
- dadurch gekennzeichnet, dass als Eingangsinformation eine Transportbedingungen während eines Transports beschreibende Bedingungsinformation verwendbar ist.

Sämtliche Ausführungen bezüglich des erfindungsgemäßen Verfahrens lassen sich analog auf die erfindungsgemäße Röntgenstrahleranordnung mit integriertem Statusüberwachungssystem übertragen.

Ein erfindungsgemäßes Computerprogramm ist beispielsweise direkt in einen Speicher einer Steuereinheit ladbar und weist Programmmittel auf, um die Schritte eines hierin beschriebenen Verfahrens auszuführen, wenn das Programm in der Steuereinheit mit einem Ermittlungsmittel und einem Verarbeitungsmittel ausgeführt wird. Das Computerprogramm, für das selbstverständlich ebenso die Ausführungen zum Verfahren fortgelten, kann auf einem erfindungsgemäßen elektronisch lesbaren Datenträger gespeichert sein, welcher mithin darauf gespeicherte elektronisch lesbare Steuerinformationen umfasst, welche zumindest ein genanntes Computerprogramm umfassen und derart ausgestaltet sind, dass sie bei Verwendung des Datenträgers in einer Steuereinheit ein hierin beschriebenes Verfahren durchführen.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: einen prinzipiellen Ablaufplan des erfindungsgemä-ßen Verfahrens,
- Fig. 2: den Aufbau einer Röntgenstrahleranordnung, und
- Fig. 3: eine Außenansicht einer Röntgenstrahleranordnung.

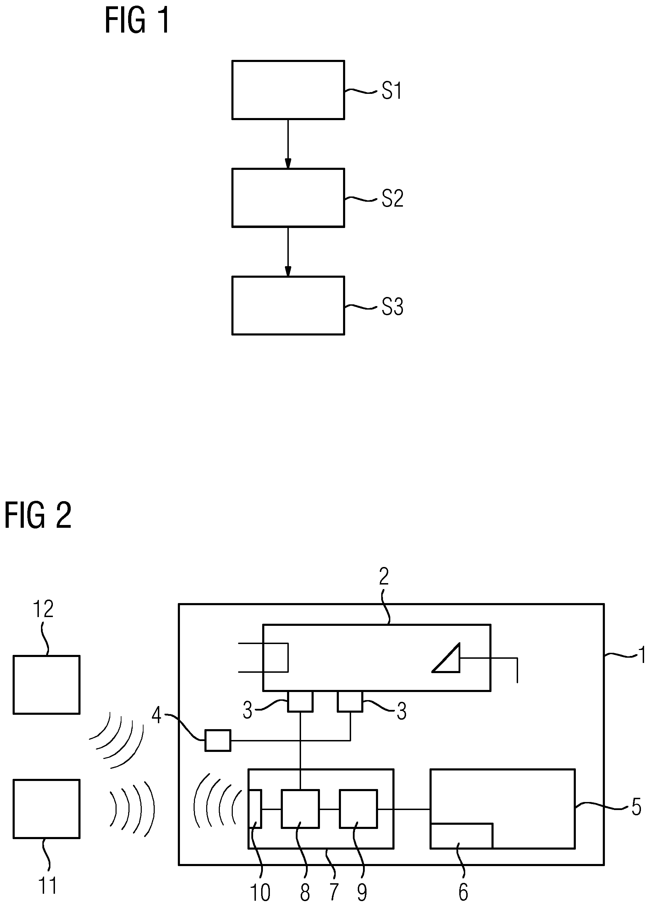

Fig. 1 illustriert den grundsätzlichen Ablauf von Ausführungsbeispielen des erfindungsgemäßen Verfahrens. Dort wird eine einer Röntgenstrahleranordnung mit einer Röntgenstrahlenquelle zugeordnete Anzeigevorrichtung, insbesondere ein Display, genutzt, um Statusinformationen auszugeben. Hierzu wird zunächst in einem Schritt S1 wenigstens ein Ermittlungsmittel genutzt, um Eingangsinformationen zu ermitteln, die sich auf die Röntgenstrahlenquelle beziehen und in ihrer Art letztlich abhängig sind von der Art der darzustellenden Statusinformation, worauf im Folgenden noch näher eingegangen werden wird. Vorliegend beschreiben die Eingangsinformationen die Nutzung der Röntgenstrahlenquelle und/oder den aktuellen Zustand der Röntgenstrahlenquelle und/oder den aktuellen Zustand der Röntgenstrahlenquelle und/oder den aktuellen Zustand der Umgebung der Röntgenstrahlungsquelle.

In einem Schritt S2 wird aus der Eingangsinformation eine an einen Benutzer auszugebende Statusinformation mittels eines Verarbeitungsmittels abgeleitet. Das bedeutet im Wesentlichen, die Eingangsinformationen, welche ja physikalischtechnische Sachverhalte beschreiben, werden unter Berücksichtigung technischer Gegebenheiten der Röntgenstrahlenquelle und gegebenenfalls der sie nutzenden Röntgeneinrichtung ausgewertet, um eine für einen Benutzer nützliche Information zu erzeugen.

Diese Statusinformation wird dann in einem Schritt S3 auf der Anzeigevorrichtung, insbesondere dem Display, angezeigt.

In bevorzugten konkreten Ausführungsbeispielen können dabei unterschiedliche Arten von Statusinformationen erzeugt und angezeigt werden, insbesondere abhängig von einer Nutzungsphase der Röntgenstrahlenquelle und/oder von Benutzereingaben. Dabei ist ohnehin anzumerken, dass den Schritten S1, S2 und S3 vorgeschaltet verschiedene Ausgabebedingungen überprüft werden können, bevor die entsprechenden Schritte S1, S2, S3 ausgeführt werden. Ausgabebedingungen können dabei selbstverständlich auch Benutzereingaben an einer der Anzeigevorrichtung zugeordneten Eingabevorrichtung, insbesondere einer in diese integrierten Eingabevorrichtung, wie bei einem Touchscreen, auswerten. Beispielsweise kann vor dem Schritt S1 eine Überprüfung erfolgen, ob grundsätzlich eine Ermittlung und Ausgabe der Statusinformation aktuell erfolgen soll/gewünscht ist/sinnvoll ist. Dies kann beispielsweise von der Nutzungsphase der Röntgenstrahleranordnung bzw. Röntgenstrahlenquelle abhängig sein, so dass beispielsweise, wenn eine Transportüberwachung stattfinden soll, anhand einer Nutzereingabe oder sonstiger vorliegender Informationen überprüft werden kann, ob gerade ein Transportvorgang vorliegt, und dergleichen. Beispielsweise kann auf der Anzeigevorrichtung auch ein Menü dargestellt werden, in dem der Nutzer zwischen unterschiedlichen Nutzungsphasen wie Endfertigung, Transport, Installation, Kalibrierung, Nutzung zur Bildgebung, Wartung, Refurbishment und dergleichen auswählen kann, woraufhin dann abhängig von dieser Auswahl unterschiedliche Statusinformationen erzeugt und angezeigt werden.

In Ausgestaltungen, in denen die Eingangsinformation mit in eine Ausgabebedingung eingehen soll, kann eine entsprechende Überprüfung auch nach dem Schritt S1 erfolgen; Ausgabebedingungen können jedoch auch nach dem Schritt S2 noch überprüft werden, beispielsweise, wenn von der Statusinformation abhängig gemacht werden soll, inwieweit eine Ausgabe der Statusinformation zweckmäßig ist. Beispielsweise sind Ausgestaltungen denkbar, in denen Warnungen nur für bestimmte Inhalte der Statusinformation auf der Anzeigevorrichtung ausgegeben werden soll.

Die Anzeigevorrichtung kann dabei auch zur Ausgabe weiterer Ausgabeinformationen neben der Statusinformation genutzt werden, wobei sich die weiteren Ausgabeinformationen bevorzugt ebenso auf die Röntgenstrahlenquelle bzw. wenigstens die Röntgenstrahleranordnung beziehen. Solche Ausgabeinformationen können insbesondere Wartungsinformationen, Gegenstandsinformationen, Herstellerinformationen, Lehrinformationen und/oder Verweise auf weitere Informationsquellen umfassen.

Bevorzugt ist wenigstens ein Display als Anzeigevorrichtung an der Röntgenstrahleranordnung selbst befestigt, beispielsweise an einem die Röntgenstrahlenquelle, welche bevorzugt eine Röntgenröhre ist, umfassenden und tragenden Gehäuse. Die entsprechende Anzeigevorrichtung zeigt dann den Status der Röntgenstrahlenquelle und verschiedene, gegebenenfalls anpassbare Optionen und/oder Betriebsmodi unterschiedliche Arten von Nutzern.

Soll in einem Ausführungsbeispiel beispielsweise eine Transportüberwachung während eines Transports der Röntgenstrahleranordnung stattfinden, wird bevorzugt eine während des Transports einsehbare Anzeigevorrichtung verwendet. Von Erfassungsmitteln der Röntgenstrahleranordnung selbst, insbesondere Sensoren, erfasste physikalische Größen wie Temperatur, Luftfeuchtigkeit, Vibrationen und dergleichen werden als Eingangsinformation ermittelt und dahingehend ausgewertet, ob die durch die Eingangsinformation beschriebenen Werte innerhalb von durch Sollwerte beschriebenen Sollbereichen liegen. Trifft dies zu, kann die entsprechende Statusinformation einfach ausgegeben werden; liegen jedoch Werte außerhalb der Sollbereiche, kann zusätzlich akustisch und/oder optisch und/oder haptisch zusätzlich ein Warnsignal gegeben werden und/oder die Statusinformation kann auffälliger wiedergegeben werden.

Eine andere konkrete, nützliche Anwendung ist die Prädiktion der voraussichtlichen zukünftigen Lebensdauer-Nutzbarkeit der Röntgenstrahlenquelle, wobei die Prädiktion dabei als Eingangsinformationen die Nutzung der Röntgenstrahlenquelle in der Vergangenheit bzw. aktuell beschreibende Nutzungsinformationen nutzen kann, wobei zudem bei der Auswertung Abnutzungsinformationen, die beispielsweise durch Tests mit gleichartigen Röntgenstrahlenquellen gewonnen wurden, herangezogen werden. Beispielsweise kann als Statusinformation dann ausgegeben werden, dass die Röntgenstrahlenquelle voraussichtlich noch für eine bestimmte Art von Untersuchungen einer bestimmten Art eingesetzt werden kann oder dergleichen.

Während einer Untersuchung kann die Anzeigevorrichtung genutzt werden, um auch Untersuchungsinformationen zur aktuellen Untersuchung auszugeben, beispielsweise ein entsprechend gewähltes Messprotokoll, ein ausgewähltes Körperteil des Patienten, der aufgenommen werden soll, und dergleichen. Hierbei ist es besonders zweckmäßig, als Statusinformation beispielsweise eine aktuelle Wärmebelastung und/oder eine Zeitdauer, für die die Röntgenstrahlenquelle für diese Untersuchung betrieben werden kann, auszugeben.

Weitere Ausgabeinformationen können in Ausführungsbeispielen auch genutzt werden, um einen Lehrbetriebsmodus umzusetzen, in dem Nutzern über entsprechend dargestellte Informationen beispielsweise Grundlagen der Röntgenphysik, Betriebsparameter der Röntgenstrahlenquelle, Funktionalitäten der Röntgenstrahlenquelle und dergleichen nähergebracht werden können.

Ein Teil der Funktionen des hier beschriebenen Statusüberwachungssystems kann auch vom Benutzer gezielt zugebucht werden, beispielsweise durch Abschluss eines entsprechenden Service-Vertrages und dergleichen. Dann kann eine entsprechende Freischaltinformation hinterlegt werden, in deren Abhängigkeit auf bestimmte freigeschaltete Services bezogene Ausgabeinformationen auf der Anzeigevorrichtung dargestellt werden können. Gerade in diesem Kontext ist es auch besonders vorteilhaft, wenn das wenigstens eine Ermittlungsmittel und/oder das Verarbeitungsmittel Zugriff auf eine drahtlose Kommunikationsschnittstelle des Statusüberwachungssystems aufweisen können, um mit externen Einrichtungen, beispielsweise einer Steuereinrichtung der Röntgeneinrichtung und/oder einer externen Servereinrichtung, insbesondere des Internets, kommunizieren zu können, sei es um Eingangsinformationen abzurufen oder aber auch sonstige Ausgabeinformationen und/oder extern auch Funktionen anzustoßen, beispielsweise bestimmte Servicedienstleistungen.

Während es im Rahmen der vorliegenden Erfindung denkbar ist, das Statusüberwachungssystem beispielsweise auf einem Mobilgerät eines Benutzers über eine Software-Applikation, mithin ein Computerprogramm, zu realisieren, das mithin sowohl das wenigstens eine Ermittlungsmittel, als auch das Verarbeitungsmittel umsetzt, wobei beispielsweise der Touchscreen des Mobilgeräts als Anzeigevorrichtung verwendet wird, ist es erfindungsgemäß bevorzugt, die Röntgenstrahleranordnung selbst mit einer Eigenintelligenz auszustatten.

Eine entsprechende erfindungsgemäße Röntgenstrahleranordnung 1 ist in ihrem schematischen Aufbau in Fig. 2 näher gezeigt. Die Röntgenstrahleranordnung 1 weist eine hier als Röntgenröhre ausgebildete Röntgenstrahlenquelle 2 auf, der vorliegend auch Erfassungsmittel 3, insbesondere Sensoren, zugeordnet sind, beispielsweise um die Temperatur, den Öldruck, Vibrationen oder dergleichen zu erfassen. Die Röntgenstrahleranordnung 1 kann auch weitere Erfassungsmittel, insbesondere Sensoren, umfassen, die beispielsweise Umgebungsbedingungen als Bedingungsinformation vermessen, beispielsweise die Luftfeuchtigkeit.

An der Röntgenstrahleranordnung 1, beispielsweise einem Gehäuse, befestigt oder zumindest der Röntgenstrahleranordnung 1 zugehörig, ist ferner eine Anzeigevorrichtung 5 mit zugeordneter oder integrierter Eingabevorrichtung 6 vorgesehen, die zur Realisierung des erfindungsgemäßen Verfahrens eingesetzt werden kann. Damit das erfindungsgemäße Verfahren vollständig durch die Röntgenstrahleranordnung 1 umgesetzt werden kann, umfasst diese ferner eine Steuereinheit 7, die sowohl das wenigstens eine Ermittlungsmittel 8 als auch das Verarbeitungsmittel 9 realisiert. Mittels einer drahtlosen Kommunikationsschnittstelle 10, beispielsweise einer Bluetooth-Schnittstelle, können Kommunikationsverbindungen zu externen Einrichtungen aufgebaut werden, wobei hier nur beispielsweise eine Steuereinrichtung 11 der Röntgeneinrichtung und eine Servereinrichtung 12, insbesondere des Internets, gezeigt sind. Über eine entsprechende Kommunikationsschnittstelle kann die Steuereinheit 7 auch mit Mobilgeräten eines Benutzers kommunizieren, beispielsweise um deren Anzeigevorrichtungen als weitere Anzeigevorrichtungen zu nutzen, wobei auch anderweitig vorgesehen sein kann, Anzeigevorrichtungen drahtlos anzusprechen, wofür bei an der Röntgenstrahleranordnung 1 verbauten Anzeigevorrichtungen 5 auch NFC-Verbindungen genutzt werden können.

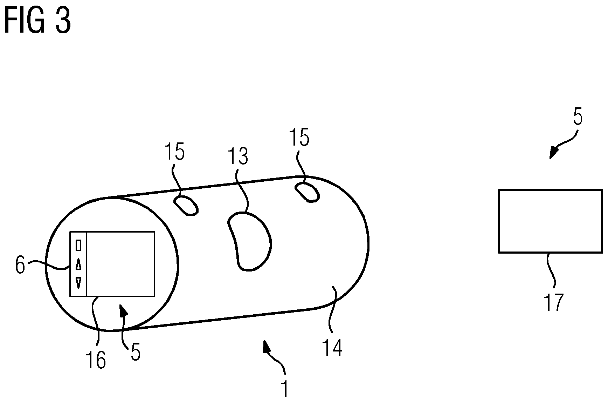

Fig. 3 zeigt eine schematische Aufsicht auf die Röntgenstrahleranordnung 1, bei der die Röntgenstrahlenquelle derart, dass nur ihr Auslass 13 sichtbar ist, in einem Gehäuse 14 verbaut ist, welches auch entsprechende Befestigungsund/oder Halterungsmittel 15 zur Befestigung der Röntgenstrahlenquelle in der Röntgeneinrichtung aufweist. Auf einer Seitenfläche ist nun die als Display 16 ausgebildete Anzeigevorrichtung 5 mit der zugeordneten, hier Bedienelemente aufweisenden Eingabevorrichtung 6 befestigt. Bei dem Display 16 kann es sich auch um einen Touchscreen handeln, bevorzugt ist zur Realisierung der Anzeigevorrichtung 5 jedoch ein Elektronisches-Papier-Display (EPD) vorgesehen, welches elektronische Tinte nutzen kann. Derartige EPDs weisen einen minimalen oder bei Nichtbetrieb gar keinen Leistungsbedarf auf.

Fig. 3 zeigt auch eine weitere, über die Kommunikationsschnittstelle 10 ansprechbare Anzeigevorrichtung 5, beispielsweise ein LCD-Display 17, welches an einer Transportverpackung oder dergleichen angeordnet werden kann, um die bereits beschriebene Transportüberwachung zu realisieren. Gegebenenfalls können auch weitere Anzeigevorrichtungen 5, die nicht zwangsläufig wie das LCD-Display 17 der Röntgenstrahleranordnung 1 zugehörig sein müssen, über die Kommunikationsschnittstelle 10 angesprochen werden.

Das hier beschriebene Ausführungsbeispiel der Röntgenstrahleranordnung 1 ermöglicht es im Übrigen auch, das Display 16 zu nutzen, um darauf einen maschinenlesbaren Code auszugeben, vorliegend einen QR-Code, der auf eine externe Informationsquelle im Internet, beispielsweise auf eine Webseite mit weiteren nützlichen Informationen zur Röntgenstrahleranordnung 1 bzw. deren Wartung, verweist. Beispielsweise kann so auf einfache Weise ein Benutzer, der mittels eines Mobilgeräts den angezeigten maschinenlesbaren Code ausliest, zu einer externen Informationsquelle weitergeleitet werden, auf der beispielsweise weitere Informationen zu Installationen, Wartung und dergleichen verfügbar sind.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Statusüberwachung einer eine Röntgenstrahlenquelle (2) umfassenden Röntgenstrahleranordnung (1) für eine Röntgeneinrichtung, umfassend folgende Schritte:
- Ermitteln einer den aktuellen Zustand der Röntgenstrahlenquelle (2) beschreibenden Eingangsinformation durch wenigstens ein Ermittlungsmittel (8),
- Ableiten einer an einen Benutzer auszugebenden Statusinformation unter Verwendung der Eingangsinformation mittels eines Verarbeitungsmittels (9), und
- Anzeigen der Statusinformation auf einer Anzeigevorrichtung (5), insbesondere einem Display (16),
**dadurch gekennzeichnet, dass** als Eingangsinformation eine Transportbedingungen während eines Transports beschreibende Bedingungsinformation verwendet wird.

2. Verfahren nach Anspruch 1, **dadurch gekennzeichnet, dass** die Eingangsinformation wenigstens teilweise durch wenigstens ein der Röntgenstrahleranordnung (1) zugeordnetes oder dieser zugehöriges Erfassungsmittel (3, 4), insbesondere einen Sensor, erfasst wird.

3. Verfahren nach Anspruch 1 oder 2, **dadurch gekennzeichnet, dass** als Eingangsinformationen zusätzlich eine die Nutzung der Röntgenstrahlenquelle (2) in der Vergangenheit und/oder für eine unmittelbar bevorstehende Messung beschreibende Nutzungsinformation und/oder eine das Alter der Röntgenstrahlenquelle (2) beschreibende Altersinformation und/oder Lagerungsbedingungen beschreibende Bedingungsinformation verwendet werden.

4. Verfahren nach einem der vorangehenden Ansprüche, **dadurch gekennzeichnet, dass** als Statusinformation eine Transportüberwachungsinformation ermittelt wird.

5. Verfahren nach Anspruch 4, **dadurch gekennzeichnet, dass** die Ermittlung der Transportüberwachungsfunktion den Vergleich der Eingangsinformationen mit Sollwerten für den Transport umfasst, wobei bei außerhalb eines durch Sollwerte beschriebenen Sollbereichs liegenden Transportbedingungen die Statusinformation als eine Warnmeldung ausgegeben wird.

6. Verfahren nach einem der vorangehenden Ansprüche, **dadurch gekennzeichnet, dass** in Abhängigkeit von einer Benutzereingabe über eine der Anzeigevorrichtung (5) und/oder der Röntgenstrahleranordnung (1) und/oder der Röntgeneinrichtung zugeordnete und/oder mit der Anzeigevorrichtung (5) in Baueinheit realisierte Eingabevorrichtung (6) und/oder von der Erfüllung einer insbesondere wenigstens eine Eingabeinformation auswertenden Ausgabebedingung die Statusinformation ausgegeben wird.

7. Verfahren nach Anspruch 6, **dadurch gekennzeichnet, dass** zusätzlich und/oder nach einer Benutzereingabe und/oder Erfüllung einer weiteren Ausgabebedingung wenigstens eine weitere, auf die Röntgenstrahlenquelle (2) bezogene, insbesondere ebenso abhängig von der Eingangsinformation ermittelte Ausgabeinformation auf der Anzeigevorrichtung (5) ausgegeben wird.

8. Verfahren nach einem der vorangehenden Ansprüche, **dadurch gekennzeichnet, dass** eine an oder benachbart der Röntgenstrahleranordnung (1) befestigte und/oder dieser zugehörige Anzeigevorrichtung (5), insbesondere ein Display (16), verwendet wird.

9. Verfahren nach Anspruch 8, **dadurch gekennzeichnet, dass** als Anzeigevorrichtung (5) elektronisches Papier verwendet wird und/oder als Statusinformation und/oder als weitere Ausgabeinformation ein maschinenlesbarer Code, der auf eine andere Informationsquelle, insbesondere im Internet, verweist, ausgegeben wird.

10. Verfahren nach Anspruch 8 oder 9, **dadurch gekennzeichnet, dass** wenigstens teilweise auch das Ermittlungsmittel (8) und/oder das Verarbeitungsmittel (9) einen Teil der Röntgenstrahleranordnung (1) , insbesondere der Steuereinheit (7), bilden.

11. Röntgenstrahleranordnung (1), aufweisend
- eine Röntgenstrahlenquelle (2),
- eine Steuereinheit (7) mit einem Ermittlungsmittel (8) zum Ermitteln einer den aktuellen Zustand der Röntgenstrahlenquelle (2) beschreibenden Eingangsinformation und einem Verarbeitungsmittel (9) zum Ableiten einer an einen Benutzer auszugebenden Statusinformation unter Verwendung der Eingangsinformation, und
- eine von der Steuereinheit (7) ansteuerbare Anzeigevorrichtung (5) zur Anzeige der Statusinformation,
**dadurch gekennzeichnet, dass** als Eingangsinformation eine Transportbedingungen während eines Transports beschreibende Bedingungsinformation verwendbar ist.

12. Computerprogramm, welches die Schritte eines Verfahrens mit den Merkmalen des Anspruchs 1 durchführt, wenn es auf einer Steuereinheit (7) mit einem Ermittlungsmittel (8) , einem Verarbeitungsmittel und einer Anzeigevorrichtung (5) ausgeführt wird.

13. Elektronisch lesbarer Datenträger, auf dem ein Computerprogramm nach Anspruch 12 gespeichert ist.

## Claims

1. Method for monitoring the status of an X-ray tube assembly (1) for an X-ray facility, which assembly includes an X-ray source (2), comprising the following steps:
- determining input information describing the current status of the X-ray source (2) by at least one determination means (8),
- deriving status information to be issued to a user, using the input information by means of a processing means (9), and
- displaying the status information on an indicator device (5), in particular on a display (16),
**characterised in that** condition-related information describing the transportation conditions during transportation is used as input information.

2. Method according to claim 1, **characterised in that** the input information is at least sometimes acquired by at least one acquisition means (3, 4) assigned to the X-ray tube assembly (1) or pertaining thereto, in particular a sensor.

3. Method according to claim 1 or 2, **characterised in that** application-related information describing the application of the X-ray source (2) in the past and/or for a directly impending measurement and/or age-related information describing the age of the X-ray source (2) and/or condition-related information describing storage conditions is additionally used as input information.

4. Method according to one of the preceding claims, **characterised in that** transportation monitoring information is determined as status information.

5. Method according to claim 4, **characterised in that** the determination of the transportation monitoring function includes the comparison of the input information with target values for transportation, wherein the status information is issued as a warning message in the event of transportation conditions outside a target range described by target values.

6. Method according to one of the preceding claims, **characterised in that** the status information is issued as a function of a user input via an input device (6) that is assigned to the indicator device (5) and/or to the X-ray tube assembly (1) and/or to the X-ray facility and/or is incorporated in one unit with the indicator device (5), and/or is issued as a function of the fulfilment of an output condition that in particular evaluates at least one item of input information.

7. Method according to claim 6, **characterised in that**, in addition to and/or subsequent to a user input and/or fulfilment of a further output condition, at least one further item of output information that has been determined relating to the X-ray source (2) is issued on the indicator device (5), in particular likewise dependent on the input information.

8. Method according to one of the preceding claims, **characterised in that** an indicator device (5), in particular a display (16), affixed on or adjacent to the X-ray tube assembly (1) and/or pertaining thereto is used.

9. Method according to claim 8, **characterised in that** electronic paper is used as an indicator device (5) and/or a machine-readable code, which directs the user to a different information source, in particular on the internet, is issued as status information and/or as further output information.

10. Method according to claim 8 or 9, **characterised in that** at least part of the determination means (8) and/or of the processing means (9) also form part of the X-ray tube assembly (1), in particular of the control unit (7).

11. X-ray tube assembly (1), having
- an X-ray source (2),
- a control unit (7) with a determination means (8) for determining input information describing the current status of the X-ray source (2) and a processing means (9) for deriving status information to be issued to a user, utilising the input information, and
- an indicator device (5) controllable by the control unit (7) to display the status information,
**characterised in that** condition-related information describing transportation conditions during transportation can be used as input information.

12. Computer program, which carries out the steps of a method with the features of claim 1 when it is run on a control unit (7) with a determination means (8), a processing means and an indicator device (5).

13. Electronically readable data carrier on which a computer program according to claim 12 is stored.

## Revendications

1. Procédé de contrôle de l'état d'un ensemble (1) radiogène à rayons X, comprenant une source (2) de rayons X, pour un dispositif à rayons X, comprenant les stades suivants :
- la détermination par au moins un moyen (8) de détermination d'une information d'entrée décrivant l'état en cours de la source (2) de rayons X,
- la déduction, au moyen d'un moyen (9) de traitement, d'une information d'état à donner à un utilisateur, en utilisant l'information d'entrée, et
- l'affichage de l'information d'état sur un dispositif (5) d'affichage, notamment sur un écran (16),
**caractérisé en ce que** l'on utilise comme information d'entrée une information de condition décrivant des conditions de transport pendant un transport.

2. Procédé suivant la revendication 1, **caractérisé en ce que** l'on détecte l'information d'entrée au moins en partie par au moins un moyen (3, 4) de détection, notamment un capteur, associé à l'ensemble (1) radiogène à rayonnement X associé ou en faisant partie.

3. Procédé suivant la revendication 1 ou 2, **caractérisé en ce que** l'on utilise comme information d'entrée en outre une information d'utilisation décrivant l'utilisation de la source (2) de rayons X dans le passé et/ou décrivant une mesure immédiatement précédente et/ou une information d'âge décrivant l'âge de la source (2) de rayons X et/ou une information de condition décrivant des conditions d'emmagasinage.

4. Procédé suivant l'une des revendications précédentes, **caractérisé en ce que** l'on détermine comme information d'état une information de contrôle de transport.

5. Procédé suivant la revendication 4, **caractérisé en ce que** la détermination de la fonction de contrôle de transport comprend la comparaison des informations d'entrée à des valeurs de consigne pour le transport, dans lequel pour des conditions de transport se trouvant en dehors d'une plage de consigne décrites par des valeurs de consigne, on émet l'information d'état sous la forme d'un message d'alerte.

6. Procédé suivant l'une des revendications précédentes, **caractérisé en ce que**, en fonction d'une entrée d'utilisateur on émet l'information d'état par l'intermédiaire d'un dispositif (6) d'entrée associé au dispositif (5) d'affichage et/ou à l'ensemble (1) radiogène à rayonnement X et/ou au dispositif à rayons X et/ou réalisé en une unité de construction avec le dispositif (5) d'affichage et/ou par la réalisation d'une condition de sortie exploitant au moins une information d'entrée.

7. Procédé suivant la revendication 6, **caractérisé en ce qu'**en plus et/ou après une entrée d'utilisateur et/ou la satisfaction d'une autre condition de sortie, on émet sur le dispositif (5) d'affichage au moins une autre information de sortie rapportée à la source (2) de rayons X, notamment déterminée également en fonction de l'information d'entrée.

8. Procédé suivant l'une des revendications précédentes, **caractérisé en ce que** l'on utilise un dispositif (5) d'affichage, notamment un écran (16) fixé sur ou au voisinage de l'ensemble (1) radiogène à rayonnement X et/ou en faisant partie.

9. Procédé suivant la revendication 8, **caractérisé en ce que** l'on utilise comme dispositif (5) d'affichage du papier électronique et/ou **en ce que** l'on émet comme information d'état et/ou comme autre information de sortie un code déchiffrable par ordinateur qui renvoie à une autre source d'information, notamment dans l'internet.

10. Procédé suivant la revendication 8 ou 9, **caractérisé en ce qu'**au moins en partie également le moyen (8) de détermination et/ou le moyen (9) de traitement forme une partie de l'ensemble (1) radiogène à rayonnement X, notamment de l'unité (7) de commande.

11. Ensemble (1) radiogène à rayons X , comportant
- une source (2) de rayons X ,
- une unité (7) de commande comprenant un moyen (8) de détermination pour la détermination d'une information d'entrée décrivant l'état en cours de la source (2) de rayons X et un moyen (9) de traitement pour la déduction en utilisant l'information d'entrée d'une information d'état à donner à un utilisateur, et
- un dispositif (5) d'affichage, pouvant être commandé par l'unité (7) de commande, pour l'affichage de l'information d'état, **caractérisé en ce que** peut être utilisée comme information d'entrée une information de condition décrivant des conditions de transport pendant un transport.

12. Programme d'ordinateur, qui effectue les stades d'un procédé ayant les caractéristiques de la revendication 1, lorsqu'il est réalisé sur une unité (7) de commande ayant un moyen (8) de détermination, un moyen de traitement et un dispositif (5) d'affichage.

13. Support de données déchiffrables électroniquement, sur lequel est mis en mémoire un programme d'ordinateur suivant la revendication 12.
